Europäisches Patentamt

**European Patent Office**

**Office européen des brevets**

(11) Publication number: **0 340 175**
**A2**

## (12) EUROPEAN PATENT APPLICATION

(21) Application number: **89830102.3**

(22) Date of filing: **07.03.89**

(51) Int. Cl.⁴: **A 61 F 2/34**

(30) Priority: **29.04.88 IT 2040088**

(43) Date of publication of application:
**02.11.89 Bulletin 89/44**

(84) Designated Contracting States:
**AT BE CH DE ES FR GB GR LI NL SE**

(71) Applicant: **G. CREMASCOLI S.p.A.**
**Via Clemente Prudenzio, 14/16**
**I-20138 Milano (IT)**

(72) Inventor: **Cremascoli, Patrizio G. Cremascoli S.p.A.**
**Via Clemente Prudenzio, 14/16**
**I-20138-Milano (IT)**

(74) Representative: **Cicogna, Franco**
**Ufficio Internazionale Brevetti Dott.Prof. Franco Cicogna**
**Via Visconti di Modrone, 14/A**
**I-20122 Milano (IT)**

(54) Hip cotyl with means for properly arranging it in the acetabulum cavity.

(57) The present invention relates to a hip cotyl, with means for properly arranging it in the acetabulum cavity,comprising a spherical body (1) having a plurality of openings (2) provided,on the convex surface of the body (1),with projecting lugs (3) to be anchored in the acetabulum cavity,on the concave surface of the body there being further applied a polyethylene insert (22) adapted to operate as a contact surface for the rotation of the head portion of a stem to be inserted into the femur.

FIG. 2

EP 0 340 175 A2

**Description**

## HIP COTYL WITH MEANS FOR PROPERLY ARRANGING IT IN THE ACETABULUM CAVITY

### BACKGROUND OF THE INVENTION

The present invention relates to a hip cotyl, provided with means for properly arranging or locating it in the acetabulum cavity.

As is known a problem associated with the reduction of fractures of the head of the femur,is that of recovering perfect mutual rolling characteristics between the head of the femur and the acetabulum cavity.

### SUMMARY OF THE INVENTION

The aim of the present invention is to provide such a hip cotyl which can be introduced,in a very simple and quick way,into the acetabulum cavity so as to firmly held it in its proper position without any risk of rejection and breakage phenomena.

Within the scope of the above mentioned aim,a main object of the present invention is to provide such a hip cotyl which affords the possibility of obtaining a perfect articulation with the head of the femur.

Another object of the present invention is to provide such a hip cotyl which is very simple construction wise and can be made by simple methods.

According to one aspect of the present invention, the above mentioned aim and objects,as well as yet other objects,which will become more apparent hereinafter,are achieved by a hip cotyl,provided with means for properly arranging said cotyl in an acetabulum cavity,characterized in that said hip cotyl comprises a partially spherical body having a plurality of openings provided,on the convex surface of said body, with projecting lugs to be anchored in said acetabulum cavity,on the concave surface of said body there being moreover applied a polyethylene insert adapted to operate as a contact surface for the rotation of a head portion of a stem member adapted for insertion into the femur.

### BRIEF DESCRIPTION OF THE DRAWINGS

Further characteristics and advantages of the invention will become more apparent from the following detailed description of a preferred,though not exclusive,embodiment thereof,which is illustrated, by way of an indicative but not limitative example,in the figures of the accompanying drawings,where:

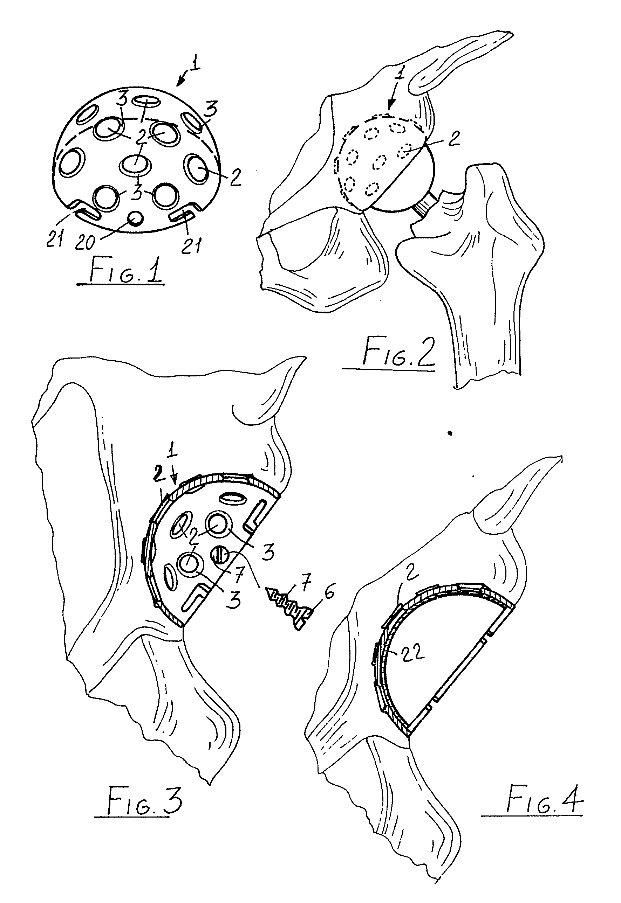

figure 1 is a schematic perspective view of the hip cotyl according to the present invention;

figure 2 is a schematic view showing the hip cotyl of the invention applied in the pelvis bone;

figure 3 is a cross-sectional view of the hip cotyl according to the invention;

and
figure 4 is another cross-section showing the subject hip cotyl applied to the pelvis bone

### DESCRIPTION OF THE PREFERRED EMBODIMENT

With reference to the figures of the accompanying drawings,the hip cotyl,provided with means for properly arranging it in an acetabulum cavity,according to the present invention,comprises a semispherical shaped body 1,made,for example,of titanium alloys ( $Ti_6$ $Al_4$).

This body 1,in particular,is provided with a plurality of throughgoing openings,indicated overally at the reference number 2,which are suitably arranged and have the feature of being provided,on the convex surface of the body 1,with projecting edges or lugs,indicated at 3,providing corresponding lug members having a respective tapering free edge.

The function of these lugs is very important, since they have been specifically designed for pressure fitting so as to directly contact the cavity of the bone wall which defines the acetabulum cavity,so as to provide an accurate positioning and anchoring inside said acetabulum cavity.

Advantageously,the openings 2 are provided with tapering portions 5 adapted to receive the heads 6 of screws 7 to be applied at different positions so as to provide a precise contact and a firm coupling.

Moreover,at least some of the lugs 3 are provided with projecting drawn edges of greater length or radial extension.More specifically there are usually provided three greater extension lugs which are arranged, in the zone of the acetabulum cavity of the pelvis bone, at points of greater strength,so as to provide a greater anchoring surface.

At the free edge portion of the shaped body 1,there are advantageously provided two or more holes 20,which can be engaged by surgical tools as the cotyl is applied to a patient,so as to affords the possibility of easily engaging or gripping the shaped body 1 to properly arrange it in the inside of the acetabulum cavity.

As is shown,at said free edge portion of the body 1,there are moreover provided tabs 21 adapted to provide a bayonet coupling,said tabs or legs being slightly slanted and so designed as to operate as an anchoring member for an insert 22,arranged at the concave surface of the body 1.

This insert is advantageously made of polyethylene and defines the contact surface thereagainst must rotate the spherical head of a femural stem member to be introduced into the femur bone of the patient.

Thus,a friction surface between the head of the femur and the hip cotyl will be formed which is provided with a very low friction coefficient.

From the above disclosure it should be apparent that the invention fully achieves the intended aim and objects.

In particular, the fact is to be pointed out that the particular shape of the shaped body 1 affords the possibility of precisely arranging and anchoring it in the hip cotyl, with a firm consequent anchoring in the acetabulum cavity.

Moreover the use of the bone screws to be engaged in the preformed openings 2 further improves the anchoring and, moreover, provides a pressure which facilitates the insertion of the several lugs into the bone wall of the acetabulum cavity.

In this connection, it should be moreover apparent that the disclosed cotyl is already ready for the application of the inner polyethylene liner, thereby facilitating all of the operations for applying said liner.

While the invention has been disclosed and illustrated with reference to a preferred embodiment thereof, it should be apparent that the disclosed embodiment would be susceptible to several modifications and variations all of which will come within the spirit and scope thereof, as defined in the appended claims.

## Claims

1- A hip cotyl, provided with means for properly arranging said cotyl in an acetabulum cavity, characterized in that said hip cotyl comprises a partially spherical body having a plurality of openings provided, on the convex surface of said body, with projecting lugs to be anchored in said acetabulum cavity, on the concave surface of said body there being moreover applied a polyethylene insert adapted to operate as a contact surface for the rotation of a head portion of a stem member adapted for insertion into the femur bone of a patient.

2- A hip cotyl according to claim 1, characterized in that said lugs have a substantially cylindrical shape, with a tapering edge.

3- A hip cotyl according to one or more of the preceding claims, characterized in that at least some of said lugs have a greater length or radial extension to be arranged at the points of said acetabulum cavity having a greater strength.

4- A hip cotyl according to one or more of the preceding claims, characterized in that said cotyl comprises, at the free edge portion of said body, a plurality of throughgoing holes to be engaged by surgical tools to insert said cotyl into the pelvis bone of said patient.

5- A hip cotyl, according to one or more of the preceding claims, characterized in that, at said free edge portion of said body, there are provided tabs defining a bayonet coupling member for coupling said insert.

6- A hip cotyl, according to one or more of the preceding claims, characterized in that said openings formed through the concave surface of said body have tapering portions for receiving the head portions of bone screws for anchoring said body in said acetabulum cavity.

7- A hip cotyl, according to one or more of the proceding claims, characterized in that said body is made of a titanium alloy ($Ti_6 Al_4$).

EP 0 340 175 A2

FIG. 1

FIG. 2

FIG. 3

FIG. 4